# EUROPEAN PATENT SPECIFICATION

(11) **EP 4 190 276 B1**
(45) Date of publication and mention of the grant of the patent: **15.10.2025**
(21) Application number: 21848923.5
(22) Date of filing: 06.07.2021
(51) Int. Cl.: A61C 9/00, A61C 13/00

(54) **DIGITALIZED ORAL DATA ACQUISITION METHOD AND APPARATUS, AND DENTAL SCANNER CONTROL SYSTEM**
VERFAHREN UND VORRICHTUNG ZUR DIGITALISIERTEN ORALEN DATENERFASSUNG UND STEUERSYSTEM FÜR ZAHNSCANNER
PROCÉDÉ ET APPAREIL D'ACQUISITION DE DONNÉES ORALES NUMÉRISÉES, ET SYSTÈME DE COMMANDE DE SCANNER DENTAIRE

(30) Priority: 31.07.2020 CN 202010763543
(43) Date of publication of application: 07.06.2023
(73) Proprietor: Shining 3D Tech Co., Ltd., Hangzhou, Zhejiang 311258 (CN)
(72) Inventor: MA, Chao, Hangzhou Zhejiang 311258 (CN)
(74) Representative: Novagraaf Group
(86) International application number: PCT/CN2021/104840
(87) International publication number: WO 2022/022242

(56) References cited:
- CN-A- 105 105 857
- CN-A- 105 287 039
- CN-A- 109 091 257
- CN-A- 109 091 257
- CN-U- 210 446 984
- JP-A- 2020 058 677
- KR-B1- 102 133 002

## Description

### Technical Field

The present disclosure relates to the technical field of device processing, and more particularly relates to a method and apparatus for acquiring digital oral cavity data and a tooth scanner control system.

### Background Art

In related technologies, a means for acquiring dental cast data in the field of tooth diagnosis and treatment is gradually turned to an intraoral three-dimensional scanning technology from impression three-dimensional scanning, and the technology is another revolution of digital dental processing, and abandons a dental cast data acquiring manner via impression, cast duplication, and three-dimensional scanning, and intraoral scanning may be directly performed to acquire tooth three-dimensional data. The two steps of impression and cast duplication are omitted in process time, material cost, labor cost and model express fee needed in the above process are saved, and discomfort of customers during impression manufacturing may be avoided.

An oral cavity digital impression instrument is also called as an intraoral three-dimensional scanner and is a device which applies a probe type optical scanning head to directly scan an oral cavity of a patient and acquire three-dimensional shape and colorful texture information of surfaces of soft or hard tissues such as teeth, gums and a mucous membrane in the oral cavity. A method for the device adopts an active structured light triangular survey imaging principle, and utilizes a digital projection system for projecting an active light pattern, and a camera acquisition system processes the acquired pattern through an algorithm for three-dimensional reconstruction and splicing.

Due to a limited space in the oral cavity, a size of the probe type optical scanning head is necessarily limited, and accordingly, it is decided that the size of the scanning head probing into the oral cavity may be controlled within 30 mm, and namely, images of only about one or two teeth may be acquired at a time. However, data of all teeth and gums in the oral cavity need to be acquired during dental designing and making in dentistry, and thus tooth image data acquired each time needs to be spliced into all three-dimensional space data consistent to an interior of the oral cavity. To improve scanning and splicing efficiency, on one hand, single-time data scanning efficiency, namely single-time scanning time and single-time scanning integrity need to be improved. On the other hand, splicing smoothness, namely a splicing success rate and splicing time need to be completed.

Cases usually needing the intraoral scanner for scanning include: repair, orthodontics and implant. In the field of implant cases, the intraoral scanner needs to acquire data of intraoral teeth of the patient and meanwhile may directly scan a scanning rod, an abutment, and the like at a missing tooth position, and materials of the scanning rod, the abutment, and the like include highly-reflective metal, bright-white materials, a titanium alloy, and the like. The scanning rod, the abutment, and the like all belong to highly-reflective objects, which bring serious trouble to the intraoral scanner utilizing an optical imaging principle. Specular reflection at different angles or light absorption phenomena will occur when light is projected to the kind of objects, which will cause an image acquisition system to only acquire an overexposed image or too dark image and import a poor image quality for three-dimensional reconstruction, and as a result, the metal scanning rod is difficult to completely scan.

Since the metal materials have more specular reflection, the intraoral scanner usually adopts a multi-angle and multi-time scanning manner when acquiring data of the kind of materials, which reduces data acquiring efficiency and makes a scanning operator and the patient uncomfortable. Particularly, when there is an optical dead angle, the intraoral scanner cannot acquire integrated data, which increases a repair workload for later design and denture wearing.

In a normal design process, intraoral three-dimensional shape data is firstly acquired and then sent to a dentistry manufacturer or a dental laboratory to design and manufacture dentures, the scanning operator will judge whether scanning is finished or not according to experience (estimated scanning data may be set to estimate denture design and manufacture, particularly metal material scanning data). When experience is insufficient or experience-based judgment is wrong, the scanning data cannot reach a denture design and manufacture standard, which will increase a complex rework process and a repeated scanning process. There are still no effective solutions for the above problems. KR 102 133 002 B1 relates to method for manufacturing dental restoration using abutment for dental digital implant.

### Summary

The present application provides a method and apparatus for acquiring digital oral cavity data and a tooth scanner control system so as to at least solve problems that in the related technologies, workers determine scanning finished according to experience, which easily causes that scanning data cannot be used and matched in a design and manufacture stage, and increases a complex rework process or a repeated scanning process.
According to one aspect of the present invention, a method for acquiring digital oral cavity data is defined in claim 1.

Optionally, scanning data of a target object is acquired based on the oral cavity scanning request includes: a scanner is controlled to perform multi-angle scanning on the target object based on the oral cavity scanning request to obtain a plurality of pieces of single scanning data of the target object, wherein at least part of single scanning data includes the scanning data of the target scanning rod, the plurality of pieces of single scanning data are spliced into integrated scanning data of the target object, and the scanning data of the target object includes the scanning data of the target scanning rod.

Optionally, the scanning data of the target scanning rod is matched with standard data of the target scanning rod includes: identification information of the target object scanning rod is acquired; the standard data of the target scanning rod is acquired based on the identification information of the target scanning rod; and the scanning data of the target scanning rod is matched with the standard data of the target scanning rod.

Optionally, the scanning data of the target scanning rod is matched with standard data of the target scanning rod includes: a scanning rod database is acquired, wherein the scanning rod database includes standard data of a plurality of types of scanning rods, the standard data of each type of scanning rods corresponds to identification information, the standard data of the plurality of types of scanning rods includes the standard data of the target scanning rod, and the standard data of the target scanning rod corresponds to identification information of the target scanning rod; the scanning rod database is loaded to a cloud server; and the standard data of the target scanning rod is downloaded from the cloud server.

Optionally, after the scanning rod database is acquired, the acquiring method further includes: the scanning rod database is loaded to a distributed memory disk of a current terminal; and the standard data of the target scanning rod is read from the distributed memory disk based on the identification information of the target scanning rod.

Optionally, the acquiring method further includes: dental cast processing data is determined based on the digital oral cavity data, wherein the dental cast processing data includes one of an abutment shape of a dental cast, a dental crown shape and a hole model target object.

According to the other aspect of the present application, a method for acquiring data is further provided, which includes: a scanning request is received; scanning data of a target object is acquired based on the scanning request, wherein the scanning data includes scanning data of an assistance part installed on the target object; the scanning data of the assistance part is matched with standard data of the assistance part; and the scanning data of the assistance part in the scanning data is replaced with the matched standard data of the assistance, and digital data of the target object is obtained.

According to another aspect of the present invention, a tooth scanner control system is defined in claim 10.

Optionally, the control system further includes: a server, which is connected to the computer and configured to store standard data of scanning rods, and send the standard data of the target scanning rod to the computer when receiving a matching request.

According to another aspect of the present invention, an apparatus for acquiring digital oral cavity data is defined in claim 12.

Optionally, the acquisition unit includes: a first control module, configured to control a scanner to perform multi-angle scanning on the target object to obtain a plurality of pieces of single scanning data of the target object based on the oral cavity scanning request, wherein at least part of single scanning data includes part of scanning data of the target scanning rod, the plurality of pieces of single scanning data are spliced into integrated scanning data of the target object, and the scanning data of the target object includes the scanning data of the target scanning rod.

Optionally, the matching unit includes: a first acquisition module, configured to acquire identification information of the target object scanning rod; a second acquisition module, configured to acquire the standard data of the target scanning rod based on the identification information of the target scanning rod; and a matching module, configured to match the scanning data of the target scanning rod with the standard data of the target scanning rod.

Optionally, the second acquisition module includes: an acquisition submodule, configured to acquire a scanning rod database, wherein the scanning rod database includes standard data of a plurality of types of scanning rods, the standard data of each type of scanning rods corresponds to identification information, the standard data of the plurality of types of scanning rods includes the standard data of the target scanning rod, and the standard data of the target scanning rod corresponds to identification information of the target scanning rod; a first loading submodule, configured to load the scanning rod database to a cloud server; and a download submodule, configured to download the standard data of the target scanning rod from the cloud server.

Optionally, the acquiring apparatus further includes: a second loading submodule, configured to load the scanning rod database to a distributed memory disk of a current terminal after the scanning rod database is acquired; and a read submodule, configured to read the standard data of the target scanning rod from the distributed memory disk based on the identification information of the target scanning rod.

Optionally, the acquiring apparatus further includes: a determine module, configured to determine dental cast processing data based on the digital oral cavity data, wherein the dental cast processing data at least includes an abutment shape of a dental cast, a dental crown shape and a hole model target object. According to another aspect of the embodiment of the present invention, an electronic device is defined in claim 13. According to another aspect of the embodiment of the present invention, a computer-readable storage medium is defined in claim 14.

In the embodiment of the present disclosure, the oral cavity scanning request is firstly received, and the scanning data of the target object is acquired based on the oral cavity scanning request, wherein the scanning data includes the scanning data of the target scanning rod installed on the target object; and then, the scanning data of the target scanning rod is matched with the standard data of the target scanning rod; and the matched standard data of the target scanning rod is utilized for replacing the scanning data of the target scanning rod in the scanning data, and the digital oral cavity data of the target object is obtained. In the embodiment, the scanning data of the scanning rod is automatically replaced with the standard data of the scanning rod in the process of acquiring the digital oral cavity data, and both the integrated scanning data and the local scanning data of the scanning rod may be replaced with the integrated standard data of the scanning rod to generate the digital oral cavity data capable of being directly designed and used, thus, after three-dimensional data is sent to a dentistry manufacturer or a dental laboratory, a designer may directly utilize the digital oral cavity data for processing the oral cavity dental cast of the user according to the standard data, which reduces a rework link and a repeated scanning process, thereby problems are solved that in the related technologies, workers determine scanning finished according to experience, which easily causes that scanning data cannot be used and matched in a design and manufacture stage, and increases a complex rework process or a repeated scanning process.

### Brief Description of the Drawings

Drawings illustrated herein are used for providing further understanding for the present disclosure and form a part of the present application, and schematic embodiments and explanations of the schematic embodiments of the present disclosure are used for explaining the present disclosure, which do not form improper limitations on the present disclosure. In the drawings:
FIG. 1 is a flowchart of an optional method for acquiring digital oral cavity data according to an embodiment of the present disclosure;
FIG. 2 is a schematic diagram of another optional tooth scanner control system according to an embodiment of the present disclosure; and
FIG. 3 is a schematic diagram of an optional apparatus for acquiring digital oral cavity data according to an embodiment of the present disclosure.

### Detailed Description

To make personnel in the technical field better understand schemes of the present disclosure, embodiments of the present disclosure are clearly and integrally described by combining drawings in the embodiments of the present disclosure as below, and it is apparent that the described embodiments are merely a part rather all embodiments of the present disclosure. Based on the embodiments of the present disclosure, all other embodiments obtained by those ordinarily skilled in the art without creative labor shall fall within the scope of protection of the present disclosure.

It needs to be explained that terms such as "first" and "second" in Description, Claims and the above drawings of the present disclosure are used for distinguishing similar objects but not necessarily used for describing specific sequences or precedence orders. It should be understood that adopted data may be exchanged under a proper situation so as to implement the embodiments, described herein, of the present disclosure in sequence except the illustrated or described sequences. In addition, terms "include" and "have" and any transformations thereof intend to cover non-exclusive inclusion, for example, a process, a method, a system, a product or a device including a series of steps or units is not limited to clearly-listed steps or units while may include unclearly-listed other steps or units or other inherent steps or units of the process, the method, the product or the device.

To facilitate those skilled in the art to understand the present disclosure, part of terms or nouns involved in the embodiments of the present disclosure are explained as below:
An oral cavity digital impression instrument is also called as an intraoral three-dimensional scanner and is a device which applies a probe type optical scanning head to directly scan an oral cavity of a patient and acquire three-dimensional shape and colorful texture information of surfaces of soft or hard tissues such as teeth, gums and a mucous membrane in the oral cavity. In the embodiment of the present disclosure, the oral cavity digital impression instrument performs imaging processing according to principles such as an active structured light triangular survey imaging principle and a confocal imaging principle which are not limited, and utilizes a digital projection system for projecting an active light pattern, and a camera acquisition system processes the acquired pattern through an algorithm for three-dimensional reconstruction and splicing.

An optical machine adopts a Digital Light Procession (DLP) shadow casting technique and uses a Digital Micromirror Device (DMD) as a key processing element to realize a digital optical processing process. Of course, the optical machine may be a laser projector or the like.

In the related technologies, when the oral cavity digital impression instrument scans the oral cavity of the patient, due to a limited space in the oral cavity, a size of the probe type optical scanning head is necessarily limited, and images of only about one or two teeth may be acquired at a time. However, data of all teeth and gums in the oral cavity need to be acquired during dental cast designing and making in dentistry, and thus a plurality of pieces of single point cloud data need to be spliced into full-mouth three-dimensional data consistent to an interior of the oral cavity. To improve scanning and splicing efficiency, on one hand, single-time data scanning efficiency, namely single-time scanning time and single-time scanning integrity need to be improved. On the other hand, splicing smoothness, namely a splicing success rate and splicing time need to be completed. In the present disclosure, a replacement action of scanned scanning rod data may be automatically finished during or after scanning, and standard data improves and ensures integrity and accuracy of the scanning rod data in a scanning stage, and reduces a rework link in the prior art.

According to the embodiment of the present disclosure, a method embodiment for acquiring digital oral cavity data is provided. It needs to be explained that steps shown in a flowchart of the drawings may be performed in a computer system with a set of computer executable instructions. In addition, although a logical sequence is shown in the flowchart, the shown or described steps may be performed in sequence different from the sequence herein under some situations.

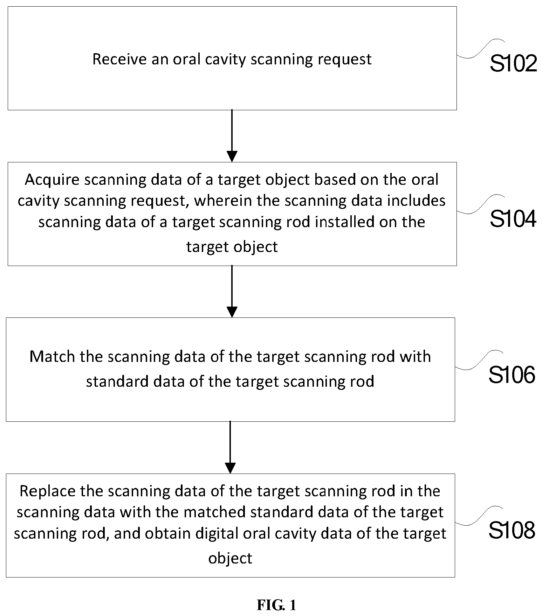

FIG. 1 is a flowchart of an optional method for acquiring digital oral cavity data according to an embodiment of the present disclosure. As shown in FIG. 1, the method includes following steps:
S102: An oral cavity scanning request is received.
S104: Scanning data of a target object is acquired based on the oral cavity scanning request, wherein the scanning data includes scanning data of a target scanning rod installed on the target object.
S106: The scanning data of the target scanning rod is matched with standard data of the target scanning rod.
S108: The matched standard data of the target scanning rod is utilized for replacing the scanning data of the target scanning rod in the scanning data, and digital oral cavity data of the target object is obtained.

Based on the digital oral cavity data, final oral cavity restorations of the target object are processed to make the target object wears the final oral cavity restorations, that is, to make a user wears the final oral cavity restorations.

By means of the above steps, the oral cavity scanning request is received; the scanning data of the target object is acquired based on the oral cavity scanning request, wherein the scanning data includes the scanning data of the target scanning rod installed on the target object; then, the scanning data of the target scanning rod is matched with the standard data of the target scanning rod; and the matched standard data of the target scanning rod is utilized for replacing the scanning data of the target scanning rod in the scanning data, and the digital oral cavity data of the target object is obtained. In the embodiment, the scanning data of the scanning rod is automatically replaced with the standard data of the scanning rod in the process of acquiring the digital oral cavity data, and both the integrated scanning data and the local scanning data of the scanning rod may be replaced with the integrated standard data of the scanning rod to generate the digital oral cavity data capable of being directly designed and used. Thus, after three-dimensional data is sent to a dentistry manufacturer or a dental laboratory, a designer may directly utilize the digital oral cavity data for processing the oral cavity dental cast of the user according to the standard data, which reduces a rework link and a repeated scanning process, thereby problems are solved that in the related technologies, workers determine scanning finished according to experience, which easily causes that scanning data cannot be used and matched in a design and manufacture stage, and increases a complex rework process or a repeated scanning process.

In the embodiment of the present disclosure, cases usually needing an intraoral scanner for scanning include: repair, orthodontics and implant, the embodiment of the present disclosure explains implant cases exemplarily. In the field of the implant cases, the scanner may directly scan various scanning rods, abutments, and the like while acquiring data of intraoral teeth of a patient. The scanning rod involved in the embodiment of the present disclosure refers to a precision assembly used for determining a location and a direction of an oral cavity implant, and the scanning rod is detachably installed in the oral cavity implant (namely, the implant). The intraoral scanner acquires three-dimensional data of the scanning rod so as to determine the location and the direction of the oral cavity implant, thereby an individual abutment is designed. The oral cavity implant (namely, the implant) is also called as a dental implant and an artificial tooth root, which is implanted into an upper jawbone or a lower jawbone of a tooth missing portion of the human body through surgery, and after surgical wound healing, an apparatus for repairing a false tooth is installed at an upper portion of the artificial tooth root; and the implant abutment (namely, the abutment) is an assistance device for the implant in dental prosthesis, and after the implant is implanted during surgery, the abutment is attached to the implant for a long time due to surgery. The abutment stretches to an outer portion of a gum to form a gum penetrating component for fixing a false tooth or other correctives (restorations). When the implant includes a complete gum penetrating part, the abutment is not needed. For false tooth scanning, the oral cavity implant is replaced with a substitution to be installed on a dental cast.

Two scanning manners are involved in the embodiment of the present disclosure to be explained, which include intraoral scanning and false tooth scanning, wherein the intraoral scanning means that the scanner probes into the oral cavity of the user and obtains scanning data after scanning the installed scanning rod, and the false tooth scanning means that a false tooth is obtained by subjecting the user's dental impression through a soft object, and accordingly scanning data is obtained by externally scanning the false tooth. The following embodiment of the present disclosure performs schematic explanation by intraoral scanning, and meanwhile is also applicable to false tooth scanning.

The above steps are combined to describe the present disclosure in detail below.

S102: An oral cavity scanning request is received.

The oral cavity scanning request may refer to a scanning instruction, and the scanning instruction may be directly generated after an order request is received. A scanning rod number may be directly selected when the order request is received, and thus, the scanning rod number may be directly obtained based on the oral cavity scanning request. After the oral cavity scanning request is received, standard data of a scanning rod is downloaded through background communication.

S104: Scanning data of a target object is acquired based on the oral cavity scanning request, wherein the scanning data includes scanning data of a target scanning rod installed on the target object.

For intraoral scanning, the scanning data includes scanning data of a tooth, a gum and a scanning rod. For false tooth scanning, the scanning data includes scanning data of a tooth model, a gum model and a scanning rod. However, since overall scanning data of three-dimensional scanning is formed by splicing a plurality of pieces of single scanning data, single scanning data of intraoral scanning may include scanning data of one or two or three of the tooth, the gum and the scanning rod, and the scanning data may be local scanning data and may also be integrated scanning data; and single scanning data of false tooth scanning may include scanning data of one or two or three of the tooth model, the gum model and the scanning rod, and the scanning data may be local scanning data and may also be integrated scanning data.

Optionally, the scanning data of the target object is acquired based on the oral cavity scanning request includes: a scanner is controlled to perform multi-angle scanning on the target object to obtain a plurality of pieces of single scanning data of the target object, wherein at least part of single scanning data includes part of scanning data of the target scanning rod, the plurality of pieces of single scanning data are spliced into the integrated scanning data of the target object, and the integrated scanning data of the target object includes the scanning data of the target scanning rod. It needs to be explained that acquiring the scanning data of the target object may be understood as acquiring the single scanning data of the target object and may also be understood as acquiring and splicing the plurality of pieces of single scanning data of the target object to obtain the integrated scanning data of the target object; at least part of single scanning data including the scanning data of the target scanning rod may be understood as including partial scanning data or integrated scanning data of the target scanning rod; and the integrated scanning data of the target object including the scanning data of the target scanning rod may be understood as including partial scanning data or integrated scanning data of the target scanning rod.

For intraoral scanning, the scanning data of the target object is acquired via splicing during real-time scanning, and namely, single scanning data acquired every time will be spliced with previous scanning data, and current scanning data of the target object is formed by splicing all scanning data acquired at the current time and before the current time. The scanning data in the embodiment refers to point cloud data, and a camera of the scanner collects 2D images, and the 2D images are subjected to three-dimensional reconstruction to obtain the point cloud data (namely, 3D images). Specifically, first single scanning data of the target object is acquired at the current time and before the current time, and current scanning data of the target object includes the first single scanning data of the target object; second single scanning data of the target object is acquired at the current time, the first single scanning data of the target object is acquired before the current time, and current scanning data of the target object is formed by splicing the first single scanning data and the second single scanning data of the target object; and Zth (Z is an integer greater than 1) single scanning data of the target object is acquired at the current time, (Z-1)th single scanning data of the target object is acquired before the current time, current scanning data of the target object is formed by splicing the Zth single scanning data of the target object and the previous (Z-1)th single scanning data. Of course, a situation that part of single scanning data of the target object fails in splicing is not excluded till scanning is finished.

Before scanning, the scanning rod is installed on the target object, the number of the scanning rod is consistent to the scanning rod number selected when the order request is received, namely, the scanning rods are the same in specification.

S106: The scanning data of the target scanning rod is matched with standard data of the target scanning rod.

The scanning data of the target scanning rod in the current scanning data and the standard data are subjected to feature splicing matching, and the standard data is subjected to transformation of coordinates in a feature splicing process.

Matching involved in the embodiment is periodical matching, particularly periodical matching in a real-time scanning process, a certain quantity of scanning data is acquired every time in the real-time scanning process and the standard data are subjected to feature splicing, or the current scanning data and the standard data are subjected to feature splicing at interval. Thus, on one hand, a pose of the standard data of the scanning data is updated and adjusted is subjected to feature splicing; and on the other hand, standard data of additional scanning rods is updated. For example, scanning data at the first time of the target object includes M (M is an integer greater than 0) pieces of single scanning data but does not include the scanning data of the target scanning rod, the standard data of the target scanning rod and the scanning data at the first time of the target object are subjected to feature splicing matching, splicing fails at the time, and only the scanning data at the first time of the target object be displayed in a scanned image at the first time ; scanning data at the second time of the target object includes N (N is an integer greater than 0) pieces of single scanning data and includes scanning data A (features are not obvious and/or a data volume is small) of a first target scanning rod, standard data of the first target scanning rod and the scanning data at the second time of the target object are subjected to feature splicing, the standard data of the first target scanning rod and the scanning data A of the first target scanning rod fail in feature splicing matching at the time, and only the scanning data at the second time of the target object is displayed in a scanned image at the second time; scanning data at the third time of the target object includes P (P is an integer greater than 0) pieces of single scanning data and includes scanning data B (features are obvious and/or a data volume is sufficient) of the first target scanning rod, the standard data of the first target scanning rod and the scanning data at the third time of the target object are subjected to feature splicing matching, and the standard data of the first target scanning rod and the scanning data B of the first target scanning rod succeed in splicing while a prompt is given, and the scanning data, at the third time of the target object and the scanning data B of the first target scanning rod are displayed in a scanned image at the third time ; and scanning data at the fourth time of the target object includes Q (Q is an integer greater than 0) pieces of single scanning data and includes scanning data (features are obvious and/or a data volume is sufficient) of the first target scanning rod and scanning data (features are obvious and/or a data volume is sufficient) of a second scanning rod, standard data of the target scanning rod and the scanning data at the fourth time of the target object are subjected to feature splicing matching, the standard data of the first target scanning rod and the scanning data of the first target scanning rod succeed in splicing while a prompt is given, the standard data of the second target scanning rod and the scanning data of the second target scanning rod succeed in splicing while a prompt is given, and the scanning data at the fourth time of the target object, the standard data of the first target scanning rod and the standard data of the second target scanning rod are displayed a scanned image at the fourth time . Of course, in a practical scanning process, only part of the above processes occurs. Visibly, along with increasing of the scanning data, especially increasing of the scanning data of the target scanning rod, the pose of the standard data of the target scanning rod may be constantly updated so as to improve splicing precision and a splicing success rate of the standard data of the target scanning rod and the scanning data of the target scanning rod without being limited by the number of target scanning rods, and one or more target scanning rods may be subjected to feature splicing matching.

In the embodiment of the present disclosure, the scanning data of the target scanning rod is matched with the standard data of the target scanning rod includes: the standard data of the target scanning rod is acquired; and the scanning data of the target scanning rod with the standard data of the target scanning rod are matched.

In the embodiment of the present disclosure, the scanning data of the target scanning rod is matched with the standard data of the target scanning rod includes: identification information of the target object scanning rod is acquired; the standard data of the target scanning rod is acquired based on the identification information of the target scanning rod; and the scanning data of the target scanning rod with the standard data of the target scanning rod are matched.

Optionally, after the oral cavity scanning request is received, computer-aided design (CAD) data corresponding to the identification information of the scanning rod is downloaded according to the identification information of the scanning rod. When the scanning process is turned to scanning via the scanning rod (scanning starts), a thread will be started on a software background at the same time, and a real-time splicing algorithm is applied to perform attempted splicing matching on the scanning data and the CAD data of the scanning rod. In the embodiment, the identification information is the number of the scanning rod, and the standard data of the scanning rod is the CAD data.

Meanwhile, in the embodiment of the present disclosure, a plurality of scanning rod features of the scanning data of the target scanning rod in the scanning process and oral cavity scanning rod data stored in a cloud server may be subjected to splicing matching (the splicing algorithm is applied as well, and attempted splicing matching is performed on real-time scanning data and the CAD data of the scanning rod based on the scanning rod features).

As an embodiment of the present disclosure, the step of matching scanning data of a target scanning rod with standard data of the target scanning rod includes: scanning rod database is acquired, wherein the scanning rod database includes standard data of a plurality of types of scanning rods, and the standard data of the plurality of types of scanning rods includes the standard data of the target scanning rod; the scanning rod database is loaded to a cloud server; and the standard data of the target scanning rod is downloaded from the cloud server.

As an embodiment of the present disclosure, the step of matching scanning data of a target scanning rod with standard data of the target scanning rod includes: a scanning rod database is acquired, wherein the scanning rod database includes standard data of a plurality of types of scanning rods, the standard data of each type of scanning rods corresponds to identification information, the standard data of the plurality of types of scanning rods includes the standard data of the target scanning rod, and the standard data of the target scanning rod corresponds to identification information of the target scanning rod; the scanning rod database is loaded to a cloud server; and the standard data of the target scanning rod is downloaded from the cloud server based on the identification information of the target scanning rod.

In the embodiment of the present disclosure, data of all types of oral cavity scanning rods may be acquired in advance and stored in a scanning rod database, and the scanning rod database is loaded to a cloud server (due to the large database, more local space is prevented from being occupied).

Of course, the scanning rod database may also be loaded to a distributed memory disk of a current terminal, and the standard data of the scanning rods is read from the distributed memory disk. Furthermore, the scanning rod database is loaded to the distributed memory disk of the current terminal, and the standard data of the target scanning rod is read from the distributed memory disk based on the identification information of the target scanning rod.

Optionally, after scanning rod data splicing matching succeeds, the user may be prompted via audio or characters.

In the embodiment of the present disclosure, during matching, due to fragment and long-time scanning, there are two matching manners: matching in a scanning process and matching after scanning.

In the first manner of matching in the scanning process, splicing matching is performed according to scanning rod features, and scanning and matching may be performed at the same time. Specifically, a certain quantity of scanning data acquired every time and the standard data of the target scanning rod are subjected to splicing matching, or the current scanning data formed by splicing all scanning data acquired at present and the standard data of the target scanning rod are subjected to splicing matching at interval. By means of the matching manner, whether the scanning data of the target scanning rod and the standard data of the target scanning rod perform correct splicing matching or not may be determined in the scanning process. When splicing matching fails or splicing matching accuracy is insufficient, the target scanning rod may be further scanned and the scanning data of the target scanning rod is increased until splicing matching succeeds, and splicing matching may succeed commonly without performing complete scanning on the target scanning rod. Thus, a problem that the target scanning rod is difficult to scan due to high reflectivity is solved. Overall scanning speed is high, data replacement is finished after the scanning data is integrally acquired and feature splicing matching of the standard data of the target scanning rod is finished, and then, the digital oral cavity data of the target object is obtained.

In the second manner of matching after scanning, unified scanning rod data matching is performed according to the scanning data of the target scanning rod during scanning after all scanning data of the user is scanned. The matching manner has advantages that all scanning rod data features may be obtained at a time, a matching result is relatively accurate, but the scanning rod needs to be scanned as completely as possible, which needs to spend long time, and when the scanning rod is incompletely scanned, a situation of unable splicing matching may happen, and as a result, scanning may be performed again.

S108: The matched standard data of the target scanning rod is utilized for replacing the scanning data of the target scanning rod in the scanning data, and digital oral cavity data of the target object is obtained.

Optionally, after scanning, the scanning data of the target scanning rod is deleted while the standard data of the target scanning rod is reserved, and namely, the digital oral cavity data of the target object includes tooth scanning data, gum scanning data and scanning rod standard data.

According to the embodiment of the present disclosure, the acquiring method further includes: dental cast processing data is determined based on the digital oral cavity data, wherein the dental cast processing data includes at least one of an abutment shape of a dental cast, a dental crown shape and a hole model target object. The dental cast processing data may be sent to a processing device after being acquired, and thus, the processing device may be utilized for manufacturing an oral cavity dental cast of the user.

After the digital oral cavity data is acquired, the location and the direction of the oral cavity implant may be determined, thus, the abutment shape, the dental crown shape and the hole model target object may be designed and sent to the processing device to realize on-site processing without secondary attendance of the patient, and after the patient is scanned, the patient may try to wear the processed oral cavity dental cast in a period of time.

By means of the above embodiment, a cloud platform may be utilized for loading the scanning rod database, and after the oral cavity of the user is scanned, the scanning data of the target scanning rod is replaced with the downloaded standard data of the target scanning rod, the final digital oral cavity data for direct design is generated, a problem that the scanning rod data cannot be used is solved, more uncertain links are omitted and rework probability is reduced.

According to another aspect of the embodiment of the present disclosure, a method for acquiring data is further provided, which includes:
A scanning request is received.

Scanning data of a target object is acquired based on the scanning request, wherein the scanning data includes scanning data of an assistance part installed on the target object.

The scanning data of the assistance part is matched with standard data of the assistance part.

The matched standard data of the assistance part is utilized for replacing the scanning data of the assistance part in the scanning data, and digital data of the target object is obtained.

The above method for acquiring the data may be applied to environments of processing of various factory products such as an abutment, a dental cast and a restoration. When the target object is processed, the scanning data may be directly replaced with standard CAD data, processed products are more standard, and product parameters and product models better conform to product processing specifications. For example, in a dental cast restoration processing process, implant digital impression three-dimensional data may be directly obtained according to the replaced CAD data, and a designer directly utilizes the digital impression three-dimensional data for processing the oral cavity dental cast of the target object without splicing matching on the three-dimensional data, thereby reducing rework links and repeated scanning processes.

A specific implementation scheme in the embodiment may refer to the embodiments of the method for acquiring the digital oral cavity data.

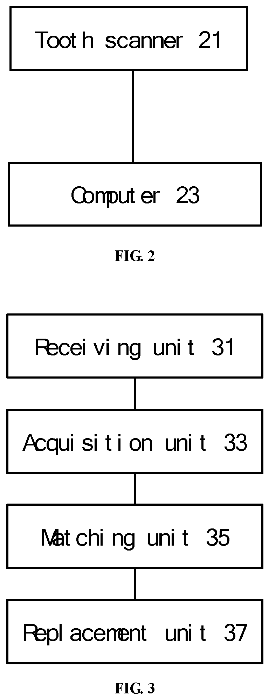

FIG. 2 is a schematic diagram of another optional tooth scanner control system according to an embodiment of the present disclosure. As shown in FIG. 2, the tooth scanner control system includes: a tooth scanner 21 and a computer 23.

The tooth scanner 21 is configured to probe into a target object for scanning so as to obtain scanning data of the target object.

The tooth scanner may be an oral cavity digital scanner, which scan an interior of an oral cavity of the target object to obtain the scanning data.

The computer 23 is connected to the tooth scanner and configured to operate programs, wherein the programs run to execute any above method for acquiring digital oral cavity data.

In the embodiment of the present disclosure, the method executed by the programs running in the computer 23 includes: an oral cavity scanning request is received; the scanning data of the target object is acquired based on the oral cavity scanning request, wherein the scanning data includes scanning data of a target scanning rod installed on the target object; the scanning data of the target scanning rod with standard data of the target scanning rod are matched; and the matched standard data of the target scanning rod is utilized for replacing the scanning data of the target scanning rod in the scanning data, and the digital oral cavity data of the target object is obtained.

Optionally, the control system further includes: a server, which is connected to the computer and configured to store standard data of scanning rods, and send the standard data of the target scanning rod to the computer when receiving a matching request.

In the embodiment of the present disclosure, the method executed by the programs running in the computer 23 further includes: the scanner to perform multi-angle scanning on the target object is controlled based on the oral cavity scanning request to obtain a plurality of pieces of single scanning data of the target object, wherein at least part of single scanning data includes scanning data of the target scanning rod, the plurality of pieces of single scanning data are spliced into integrated scanning data of the target object, and the scanning data of the target object includes the scanning data of the target scanning rod.

In the embodiment of the present disclosure, the method executed by the programs running in the computer 23 further includes: identification information of the target object scanning rod is acquired; the standard data of the target scanning rod is acquired based on the identification information of the target scanning rod; and the scanning data of the target scanning rod are matched with the standard data of the target scanning rod.

In the embodiment of the present disclosure, the method executed by the programs running in the computer 23 further includes: a scanning rod database is acquired, wherein the scanning rod database includes standard data of a plurality of types of scanning rods, the standard data of each type of scanning rods corresponds to identification information, the standard data of the plurality of types of scanning rods includes the standard data of the target scanning rod, and the standard data of the target scanning rod corresponds to identification information of the target scanning rod; the scanning rod database is loaded to a cloud server; and the standard data of the target scanning rod is downloaded from the cloud server.

According to another option, the acquiring method further includes: the scanning rod database is loaded to a distributed memory disk of a current terminal after the scanning rod database is acquired; and the standard data of the target scanning rod is read from the distributed memory disk based on the identification information of the target scanning rod.

In the embodiment of the present disclosure, the method executed by the programs running in the computer 23 further includes: dental cast processing data is determined based on the digital oral cavity data, wherein the dental cast processing data at least includes one of an abutment shape of a dental cast, a dental crown shape and a hole model target object.

According to the above tooth scanner control system, the tooth scanner 21 probes into the target object for scanning to obtain the scanning data of the target object. When the dental cast is manufactured by the computer 23, the oral cavity scanning request is firstly received, and the scanning data of the target object is acquired based on the oral cavity scanning request, wherein the scanning data includes the scanning data of the target scanning rod installed on the target object; and then, the scanning data of the target scanning rod is matched with the standard data of the target scanning rod; and the matched standard data of the target scanning rod is utilized for replacing the scanning data of the target scanning rod in the scanning data, and the digital oral cavity data of the target object is obtained. In the embodiment, the scanning data of a scanning rod area may be automatically replaced in the process of acquiring the digital oral cavity data, and the digital oral cavity data (understood as standard oral cavity implant CAD data) capable of being directly designed and used is generated. Thus, after three-dimensional data is sent to a dentistry manufacturer or a dental laboratory, a designer may directly utilize the digital oral cavity data for processing the oral cavity dental cast of the target object without splicing matching on the scanning rod standard data, thereby rework links and repeated scanning processes are reduced, and problems are solved that in related technologies, workers determine scanning finished according to experience, which easily causes that scanning data cannot be used and matched in a design and manufacture stage, and increases a complex rework process or a repeated scanning process.

A specific implementation scheme in the embodiment may refer to the embodiments of the method for acquiring the digital oral cavity data.

FIG. 3 is a schematic diagram of an optional apparatus for acquiring digital oral cavity data according to an embodiment of the present disclosure. As shown in FIG. 3, the acquiring apparatus may include: a receiving unit 31, an acquisition unit 33, a matching unit 35 and a replacement unit 37.

The receiving unit 31 is configured to receive an oral cavity scanning request.

The acquisition unit 33 is configured to acquire scanning data of a target object based on the oral cavity scanning request, wherein the scanning data includes scanning data of a target scanning rod installed on the target object.

The matching unit 35 is configured to match the scanning data of the target scanning rod with standard data of the target scanning rod.

The replacement unit 37 is configured to utilize the matched standard data of the target scanning rod for replacing the scanning data of the target scanning rod in the scanning data, and obtain the digital oral cavity data of the target object.

According to the above apparatus for acquiring the digital oral cavity data, the receiving unit 31 firstly receives the oral cavity scanning request in the process of acquiring the digital oral cavity data; the acquisition unit 33 acquires the scanning data of the target object based on the oral cavity scanning request, wherein the scanning data includes the scanning data of the target scanning rod installed on the target object; then, the matching unit 35 matches the scanning data of the target scanning rod with the standard data of the target scanning rod; and replacement unit 37 utilizes the matched standard data of the target scanning rod for replacing the scanning data of the target scanning rod in the scanning data, thereby obtaining the digital oral cavity data of the target object. In the embodiment, the scanning data of a scanning rod area may be automatically replaced in the process of acquiring the digital oral cavity data, and the digital oral cavity data (understood as standard oral cavity implant CAD data) capable of being directly designed and used is generated. Thus, after three-dimensional data is sent to a dentistry manufacturer or a dental laboratory, a designer may directly utilize the digital oral cavity data for processing an oral cavity dental cast of the target object without splicing matching on the scanning rod standard data, thereby rework links and repeated scanning processes are reduced, and problems are solved that in related technologies, workers determine scanning finished according to experience, which easily causes that scanning data cannot be used and matched in a design and manufacture stage, and increases a complex rework process or a repeated scanning process.

Optionally, the acquisition unit includes: an installation module, configured to be based on the oral cavity scanning request, and a first control module, configured to control a scanner to perform multi-angle scanning on the target object to obtain a plurality of pieces of single scanning data of the target object, wherein at least part of single scanning data includes part of scanning data of the target scanning rod, the plurality of pieces of single scanning data are spliced into integrated scanning data of the target object, and the scanning data of the target object includes the scanning data of the target scanning rod.

In the embodiment of the present disclosure, the matching unit includes: a first acquisition module, configured to acquire identification information of the target object scanning rod; a second acquisition module configured to acquire the standard data of the target scanning rod based on the identification information of the target scanning rod; and a matching module, configured to match the scanning data of the target scanning rod with the standard data of the target scanning rod.

Optionally, the second acquisition module includes an acquisition submodule, configured to acquire a scanning rod database, wherein the scanning rod database includes standard data of a plurality of types of scanning rods, the standard data of each type of scanning rods corresponds to identification information, the standard data of the plurality of types of scanning rods includes the standard data of the target scanning rod, and the standard data of the target scanning rod corresponds to identification information of the target scanning rod; a first loading submodule, configured to load the scanning rod database to a cloud server; and a download submodule, configured to download the standard data of the target scanning rod from the cloud server.

Optionally, the acquiring apparatus further includes: a second loading submodule, configured to load the scanning rod database to a distributed memory disk of a current terminal after the scanning rod database is acquired; and a read submodule, configured to read the standard data of the target scanning rod from the distributed memory disk based on the identification information of the target scanning rod.

In the embodiment of the present disclosure, the acquiring apparatus further includes: a determine module, configured to determine dental cast processing data based on the digital oral cavity data, wherein the dental cast processing data includes at least one of an abutment shape of a dental cast, a dental crown shape and a hole model target object.

The above apparatus for acquiring the digital oral cavity data may further include a processor and a memory, and the receiving unit 31, the acquisition unit 33, the matching unit 35, the replacement unit 37, and the like all serve as program units to be stored in the memory, and the processor executes the above program units stored in the memory to realize corresponding functions.

The processor includes a core, and the core calls the corresponding program units from the memory. One or more cores may be set, and based on the digital oral cavity data, the oral cavity dental cast of the target object is processed by adjusting core parameters so that the target object may wear the oral cavity dental cast.

The above memory may include a volatile memory, a RAM and/or a nonvolatile internal memory and other forms in a computer-readable medium, such as a ROM or a flash RAM, and the memory includes at least one memory chip.

According to another aspect of the embodiment of the present disclosure, an electronic device is further provided , which includes a processor; and a memory, which is configured to store executable instructions of the processor, wherein the processor is configured to execute the executable instructions to execute any above method for acquiring digital oral cavity data.

According to another aspect of the embodiment of the present disclosure, a computer-readable storage medium is further provided, which includes stored computer programs, wherein the computer programs run, a device wherein the computer-readable storage medium is located is controlled to execute any above method for acquiring digital oral cavity data.

The present application further provides a computer program product. The computer program product being executed on a data processing device is applicable to initializing programs with following method steps: an oral cavity scanning request is received; scanning data of a target object based on the oral cavity scanning request is acquired, wherein the scanning data includes scanning data of a target scanning rod installed on the target object; the scanning data of the target scanning rod is matched with standard data of the target scanning rod; and the matched standard data of the target scanning rod is utilized for replacing the scanning data of the target scanning rod in the scanning data, and the digital oral cavity data of the target object is obtained.

The serial numbers of the above embodiments of the present disclosure are merely used for descriptions instead of representing good or bad of the embodiments.

In the above embodiments of the present disclosure, a particular emphasis is placed on a description on each embodiment, and parts not described in detail in one embodiment may refer to related descriptions in other embodiments.

It is to be understood that contents disclosed by the several embodiments provided by the present application may be realized by other manners. The above-described apparatus embodiments are merely schematic, such as unit division which may be logic function division; and during practical implementation, there may be additional division manners, for example, a plurality of units or assemblies may be combined or integrated into another system, or some characteristics may be ignored or not executed. In addition, shown or discussed mutual coupling or direct coupling or communication connection may be realized through some interfaces, and unit or module indirect coupling or communication connection may be in an electrical form or other forms.

Units described as separation components may be or may be not physically separated, and components for unit display may be or may be not physical units, may be located at the same position, or may be distributed in a plurality of units. Part or all of the units may be selected according to actual demands to achieve objectives of the schemes of the embodiments.

In addition, functional units in the embodiments of the present disclosure may be integrated in one processing unit, or independently and physically exist, or two or more units may be integrated in one unit. The above integrated unit may be realized in a hardware form or a form of a software functional unit.

When the integrated unit is realized in the form of the software functional unit and serve as an independent product to be sold or used, the integrated unit may be stored in a computer-readable storage medium. Based on the understanding, the present disclosure essentially or parts making contribution to the prior art or all or part of the embodiment s may be embodied in a software product form. A computer software product is stored in a storage medium and includes a plurality of instructions for making a computer device (a personal computer, a server, or a network device, or the like) perform all or part of the steps of the methods in the embodiments of the present disclosure. The storage medium includes a U disk, a Read-Only Memory (ROM), a Random Access Memory (RAM), a mobile hard disk, a diskette or a light disk or other media capable of storing program codes.

The above contents are merely preferred implementation modes of the present disclosure. It needs to be indicated that a plurality of improvements and embellishments may be made by those ordinarily skilled in the technical field without departing from the principle of the present disclosure and should fall within the scope of protection of the present disclosure.

### Industrial applicability

The schemes provided by the embodiments of the present application may be used for acquiring the digital oral cavity data. A replacement action of the scanned scanning rod data may be automatically finished during or after scanning, and the standard data improves and ensures integrity and accuracy of the scanning rod data in the scanning stage. In the embodiments of the present application, the scanning data of the scanning rod is automatically replaced with the standard data of the scanning rod in the process of acquiring the digital oral cavity data, and both the integrated scanning data and the local scanning data of the scanning rod may be replaced with the integrated standard data of the scanning rod to generate the digital oral cavity data capable of being directly designed and used. Thus, after the three-dimensional data is sent to the dentistry manufacturer or the dental laboratory, the designer may directly utilize the digital oral cavity data for processing the oral cavity dental cast of the user according to the standard data, which reduces the rework link and the repeated scanning process, thereby the problems are solved that in the related technologies, workers determine scanning finished according to experience, which easily causes that scanning data cannot be used and matched in a design and manufacture stage, and increases a complex rework process or a repeated scanning process.

## Claims

1. A method for acquiring digital data, comprising:
receiving a scanning request;
acquiring scanning data of a target object based on the scanning request, wherein the scanning data comprises scanning data of an assistance part installed on the target object, the assistance part is a target scanning rod, and the target object is an object onto which the target scanning rod is installed;
matching the scanning data of the assistance part with standard data of the assistance part, the standard data of the assistance part is a computer-aided design data corresponding to the assistance part; and
replacing the scanning data of the assistance part in the scanning data of the target object with the matched standard data of the assistance part, and obtaining the digital data of the target object.

2. The acquiring method according to claim 1
wherein the step of acquiring scanning data of a target object based on the scanning request comprises: acquiring scanning data of a target object based on the oral cavity scanning request, wherein the scanning data comprises scanning data of the target scanning rod installed on the target object, the standard data of the target scanning rod is the design data corresponding to the target scanning rod;
wherein the step of matching the scanning data of the assistance part with standard data of the assistance part comprises: matching the scanning data of the target scanning rod with standard data of the target scanning rod; and
wherein the step of replacing the scanning data of the assistance part in the scanning data of the target object with the matched standard data of the assistance part , and obtaining the digital data of the target object comprises: replacing the scanning data of the target scanning rod in the scanning data of the target object with the matched standard data of the target scanning rod, and obtaining the digital oral cavity data of the target object.

3. The acquiring method according to claim 2, wherein the step of acquiring scanning data of a target object based on the oral cavity scanning request comprises:
controlling a scanner to perform multi-angle scanning on the target object based on the oral cavity scanning request to obtain a plurality of pieces of single scanning data of the target object, wherein at least part of single scanning data comprises the scanning data of the target scanning rod, the plurality of pieces of single scanning data are spliced into integrated scanning data of the target object, and the scanning data of the target object comprises the scanning data of the target scanning rod.

4. The acquiring method according to claim 2, wherein the step of matching the scanning data of the target scanning rod with standard data of the target scanning rod comprises:
acquiring identification information of the target object scanning rod;
acquiring the standard data of the target scanning rod based on the identification information of the target scanning rod; and
matching the scanning data of the target scanning rod with the standard data of the target scanning rod.

5. The acquiring method according to claim 4, wherein the step of acquiring identification information of the target object scanning rod comprises:
acquiring identification information of the target object scanning rod from the oral cavity scanning request.

6. The acquiring method according to claim 2, wherein the step of matching the scanning data of the target scanning rod with standard data of the target scanning rod comprises:
acquiring a certain quantity of scanning data every time in the real-time scanning process and matching current scanning with the standard data, wherein the current scanning data is formed by splicing all scanning data acquired at current time and before the current time;
or matching current scanning data and the standard data at interval in the real-time scanning process, wherein the current scanning data is formed by splicing all scanning data acquired at current time and before the current time.

7. The acquiring method according to claim 2, wherein the step of matching the scanning data of the target scanning rod with standard data of the target scanning rod comprises:
matching the scanning data of the target scanning rod in all the scanning data with the standard data of the target scanning rod after scanning all the scanning data of the target object.

8. The acquiring method according to any one of claims 2, 4, 6, 7, wherein the step of matching the scanning data of the target scanning rod with standard data of the target scanning rod comprises:
acquiring a scanning rod database, wherein the scanning rod database comprises standard data of a plurality of types of scanning rods, the standard data of each type of scanning rods corresponds to identification information, the standard data of the plurality of types of scanning rods comprises the standard data of the target scanning rod, and the standard data of the target scanning rod corresponds to identification information of the target scanning rod;
loading the scanning rod database to a cloud server; and
downloading the standard data of the target scanning rod from the cloud server.

9. The acquiring method according to any one of claims 2, 4, 6, 7, wherein the step of matching the scanning data of the target scanning rod with standard data of the target scanning rod comprises:
acquiring a scanning rod database, wherein the scanning rod database comprises standard data of a plurality of types of scanning rods, the standard data of each type of scanning rods corresponds to identification information, the standard data of the plurality of types of scanning rods comprises the standard data of the target scanning rod, and the standard data of the target scanning rod corresponds to identification information of the target scanning rod;
loading the scanning rod database to a distributed memory disk of a current terminal; and
downloading the standard data of the target scanning rod from the distributed memory disk.

10. A tooth scanner control system, comprising:
a tooth scanner (21), configured to scan a target object so as to obtain scanning data of the target object; and
a computer (23), connected to the tooth scanner (21) and configured to operate programs, wherein the programs run to execute the method for acquiring digital oral cavity data according to any one of claims 1 to 9.

11. The control system according to claim 10, further comprising:
a server, connected to the computer (23) and configured to store standard data of scanning rods, and send standard data of a target scanning rod to the computer (23) when receiving a matching request.

12. An apparatus for acquiring digital oral cavity data, comprising:
a receiving unit (31), configured to receive an oral cavity scanning request;
an acquisition unit (33), configured to acquire scanning data of a target object based on the oral cavity scanning request, wherein the scanning data comprises scanning data of a target scanning rod installed on the target object, the target object is an object onto which the target scanning rod is installed, and the standard data of the target scanning rod is a design data corresponding to the target scanning rod;
a matching unit (35), configured to match the scanning data of the target scanning rod with standard data of the target scanning rod; and
a replacement unit (37), configured to utilize the matched standard data of the target scanning rod for replacing the scanning data of the target scanning rod in the scanning data of the target object, and obtain the digital oral cavity data of the target object.

13. An electronic device, comprising:
a processor; and
a memory, configured to store executable instructions of the processor,
wherein the processor is configured to execute the executable instructions to execute the method for acquiring digital oral cavity data according to any one of claims 1 to 9.

14. A computer-readable storage medium, comprising stored computer programs, wherein when the computer programs run, a device wherein the computer-readable storage medium is located is controlled to execute the method for acquiring digital oral cavity data according to any one of claims 1 to 9.

## Patentansprüche

1. Verfahren zum Erfassen digitaler Daten, umfassend:
Empfangen einer Scananforderung;
Erfassen von Scandaten eines Zielobjekts basierend auf der Scananforderung, wobei die Scandaten Scandaten eines Hilfsteils umfassen, das an dem Zielobjekt installiert ist, das Hilfsteil ein Zielscanstab ist und das Zielobjekt ein Objekt ist, an dem der Zielscanstab installiert ist;
Abgleichen der Scandaten des Hilfsteils mit Standarddaten des Hilfsteils, wobei die Standarddaten des Hilfsteils computergestützte Entwurfsdaten sind, die dem Hilfsteil entsprechen;
und Ersetzen der Scandaten des Hilfsteils in den Scandaten des Zielobjekts mit den abgeglichenen Standarddaten des Hilfsteils und Erhalten der digitalen Daten des Zielobjekts.

2. Erfassungsverfahren nach Anspruch 1
wobei der Schritt des Erfassens von Scandaten eines Zielobjekts basierend auf der Scananforderung umfasst: Erfassen von Scandaten eines Zielobjekts basierend auf der Scananforderung für eine Mundhöhle, wobei die Scandaten Scandaten des Zielscanstabs umfassen, der an dem Zielobjekt installiert ist, wobei die Standarddaten des Zielscanstabs die Entwurfsdaten sind, die dem Zielscanstab entsprechen;
wobei der Schritt des Abgleichens der Scandaten des Hilfsteils mit Standarddaten des Hilfsteils umfasst: Abgleichen der Scandaten des Zielscanstabs mit Standarddaten des Zielscanstabs; und
wobei der Schritt des Ersetzens der Scandaten des Hilfsteils in den Scandaten des Zielobjekts durch die abgeglichenen Standarddaten des Hilfsteils und des Erhaltens der digitalen Daten des Zielobjekts umfasst: Ersetzen der Scandaten des Zielscanstabs in den Scandaten des Zielobjekts durch die abgeglichenen Standarddaten des Zielscanstabs und Erhalten der digitalen Mundhöhlendaten des Zielobjekts.

3. Erfassungsverfahren nach Anspruch 2, wobei der Schritt des Erfassens von Scandaten eines Zielobjekts basierend auf der Scananforderung für die Mundhöhle umfasst:
Steuern eines Scanners, um basierend auf der Scananforderung für die Mundhöhle einen Mehrwinkelscan des Zielobjekts durchzuführen, um eine Vielzahl von einzelnen Scandatenelementen des Zielobjekts zu erhalten, wobei mindestens ein Teil der einzelnen Scandaten die Scandaten des Zielscanstabs umfasst, die Vielzahl von einzelnen Scandaten zu integrierten Scandaten des Zielobjekts zusammengefügt werden und die Scandaten des Zielobjekts die Scandaten des Zielscanstabs umfassen.

4. Erfassungsverfahren nach Anspruch 2, wobei der Schritt des Abgleichens der Scandaten des Zielscanstabs mit Standarddaten des Zielscanstabs umfasst:
Erfassen von Identifikationsinformationen des Zielobjektscanstabs;
Erfassen der Standarddaten des Zielscanstabs basierend auf den Identifikationsinformationen des Zielscanstabs; und
Abgleichen der Scandaten des Zielscanstabs mit den Standarddaten des Zielscanstabs.

5. Erfassungsverfahren nach Anspruch 4, wobei der Schritt des Erfassens von Identifikationsinformationen des Zielobjektscanstabs umfasst:
Erfassen von Identifikationsinformationen des Zielobjektscanstabs aus der Scananforderung für die Mundhöhle.

6. Erfassungsverfahren nach Anspruch 2, wobei der Schritt des Abgleichens der Scandaten des Zielscanstabs mit Standarddaten des Zielscanstabs umfasst:
Erfassen einer bestimmten Menge von Scandaten bei jedem Echtzeitscanvorgang und Abgleichen der aktuellen Scandaten mit den Standarddaten, wobei die aktuellen Scandaten durch das Zusammenfügen aller Scandaten ausgebildet werden, die zu dem aktuellen Zeitpunkt und vor dem aktuellen Zeitpunkt erfasst werden;
oder Abgleichen der aktuellen Scandaten und der Standarddaten in Intervallen in dem Echtzeitscanvorgang, wobei die aktuellen Scandaten durch das Zusammenfügen aller Scandaten ausgebildet werden, die zu dem aktuellen Zeitpunkt und vor dem aktuellen Zeitpunkt erfasst werden.

7. Erfassungsverfahren nach Anspruch 2, wobei der Schritt des Abgleichens der Scandaten des Zielscanstabs mit Standarddaten des Zielscanstabs umfasst:
Abgleichen der Scandaten des Zielscanstabs in allen Scandaten mit den Standarddaten des Zielscanstabs nach dem Scannen aller Scandaten des Zielobjekts.

8. Erfassungsverfahren nach einem der Ansprüche 2, 4, 6, 7, wobei der Schritt des Abgleichens der Scandaten des Zielscanstabs mit Standarddaten des Zielscanstabs umfasst:
Erfassen einer Scanstabdatenbank, wobei die Scanstabdatenbank Standarddaten einer Vielzahl von Scanstabtypen umfasst, die Standarddaten jedes Scanstabtyps Identifikationsinformationen entsprechen, die Standarddaten der Vielzahl von Scanstabtypen die Standarddaten des Zielscanstabs umfassen und die Standarddaten des Zielscanstabs Identifikationsinformationen des Zielscanstabs entsprechen;
Laden der Scanstabdatenbank auf einen Cloud-Server; und
Herunterladen der Standarddaten des Zielscanstabs von dem Cloud-Server.

9. Erfassungsverfahren nach einem der Ansprüche 2, 4, 6, 7, wobei der Schritt des Abgleichens der Scandaten des Zielscanstabs mit Standarddaten des Zielscanstabs umfasst:
Erfassen einer Scanstabdatenbank, wobei die Scanstabdatenbank Standarddaten einer Vielzahl von Scanstabtypen umfasst, die Standarddaten jedes Scanstabtyps Identifikationsinformationen entsprechen, die Standarddaten der Vielzahl von Scanstabtypen die Standarddaten des Zielscanstabs umfassen und die Standarddaten des Zielscanstabs Identifikationsinformationen des Zielscanstabs entsprechen;
Laden der Scanstabdatenbank auf eine verteilte Speicherplatte eines aktuellen Endgeräts; und
Herunterladen der Standarddaten des Zielscanstabs von der verteilten Speicherplatte.

10. Zahnscannersteuersystem, umfassend:
einen Zahnscanner (21), der konfiguriert ist, um ein Zielobjekt zu scannen, um Scandaten des Zielobjekts zu erhalten; und
einen Computer (23), der mit dem Zahnscanner (21) verbunden und konfiguriert ist, um Programme zu betreiben, wobei die Programme ablaufen, um das Verfahren zum Erfassen digitaler Mundhöhlendaten nach einem der Ansprüche 1 bis 9 auszuführen.

11. Steuersystem nach Anspruch 10, ferner umfassend:
einen Server, der mit dem Computer (23) verbunden und konfiguriert ist, um Standarddaten von Scanstäben zu speichern und Standarddaten eines Zielscanstabs an den Computer (23) zu senden, wenn eine Übereinstimmungsanforderung erhalten wird.

12. Einrichtung zum Erfassen digitaler Mundhöhlendaten, umfassend:
eine Empfangseinheit (31), die konfiguriert ist, um eine Scananforderung der Mundhöhle zu empfangen;
eine Erfassungseinheit (33), die konfiguriert ist, um Scandaten eines Zielobjekts basierend auf der Scananforderung für die Mundhöhle zu erfassen, wobei die Scandaten Scandaten eines Zielscanstabs umfassen, der an dem Zielobjekt installiert ist, das Zielobjekt ein Objekt ist, an dem der Zielscanstab installiert ist, und die Standarddaten des Zielscanstabs Entwurfsdaten sind, die dem Zielscanstab entsprechen;
eine Abgleicheinheit (35), die konfiguriert ist, um die Scandaten des Zielscanstabs mit Standarddaten des Zielscanstabs abzugleichen; und
eine Ersetzungseinheit (37), die konfiguriert ist, um die abgeglichenen Standarddaten des Zielscanstabs zu verwenden, um die Scandaten des Zielscanstabs in den Scandaten des Zielobjekts zu ersetzen und die digitalen Mundhöhlendaten des Zielobjekts zu erhalten.

13. Elektronische Vorrichtung, umfassend:
einen Prozessor; und
einen Speicher, der konfiguriert ist, um ausführbare Anweisungen des Prozessors zu speichern,
wobei der Prozessor konfiguriert ist, um die ausführbaren Anweisungen auszuführen, um das Verfahren zum Erfassen digitaler Mundhöhlendaten nach einem der Ansprüche 1 bis 9 auszuführen.

14. Computerlesbares Speichermedium, umfassend gespeicherte Computerprogramme, wobei, wenn die Computerprogramme laufen, eine Vorrichtung, in der sich das computerlesbare Speichermedium befindet, gesteuert wird, um das Verfahren zum Erfassen digitaler Mundhöhlendaten nach einem der Ansprüche 1 bis 9 auszuführen.

## Revendications

1. Procédé d'acquisition de données numériques, comprenant :
la réception d'une demande de balayage ;
l'acquisition de données de balayage d'un objet cible sur la base de la demande de balayage, dans lequel les données de balayage comprennent des données de balayage d'une partie d'assistance installée sur l'objet cible, la partie d'assistance est une tige de balayage cible, et l'objet cible est un objet sur lequel la tige de balayage cible est installée ;
la mise en correspondance des données de balayage de la partie d'assistance avec des données standard de la partie d'assistance, les données standard de la partie d'assistance sont des données
de conception assistée par ordinateur correspondant à la partie d'assistance ; et le remplacement des données de balayage de la partie d'assistance dans les données de balayage de l'objet cible par les données standard mises en correspondance de la partie d'assistance, et l'obtention des données numériques de l'objet cible.

2. Procédé d'acquisition selon la revendication 1
dans lequel l'étape consistant à acquérir des données de balayage d'un objet cible sur la base de la demande de balayage comprend : l'acquisition de données de balayage d'un objet cible sur la base de la demande de balayage de cavité buccale, dans lequel les données de balayage comprennent des données de balayage de la tige de balayage cible installée sur l'objet cible, les données standard de la tige de balayage cible sont les données de conception correspondant à la tige de balayage cible ;
dans lequel l'étape consistant à mettre en correspondance les données de balayage de la partie d'assistance avec des données standard comprend la mise en correspondance des données de balayage de la tige de balayage cible avec des données standard de la tige de balayage cible ; et
dans lequel l'étape consistant à remplacer les données de balayage de la partie d'assistance dans les données de balayage de l'objet cible par les données standard mises en correspondance de la partie d'assistance et à obtenir les données numériques de l'objet cible comprend : le remplacement des données de balayage de la tige de balayage cible dans les données de balayage de l'objet cible par les données standard mises en correspondance de la tige de balayage cible, et l'obtention des données numériques de cavité buccale de l'objet cible.

3. Procédé d'acquisition selon la revendication 2, dans lequel l'étape consistant à acquérir des données de balayage d'un objet cible sur la base de la demande de balayage de cavité buccale comprend :
la commande d'un scanner pour effectuer un balayage multi-angle sur l'objet cible sur la base de la demande de balayage de cavité buccale afin d'obtenir une pluralité de morceaux de données de balayage unique de l'objet cible, dans lequel au moins une partie des données de balayage unique comprend les données de balayage de la tige de balayage cible, la pluralité de morceaux de données de balayage unique sont assemblées dans des données de balayage intégrées de l'objet cible, et les données de balayage de l'objet cible comprennent les données de balayage de la tige de balayage cible.

4. Procédé d'acquisition selon la revendication 2, dans lequel l'étape consistant à mettre en correspondance les données de balayage de la tige de balayage cible avec des données standard de la tige de balayage cible comprend :
l'acquisition d'informations d'identification de la tige de balayage d'objet cible ;
l'acquisition des données standard de la tige de balayage cible sur la base des informations d'identification de la tige de balayage cible ; et
la mise en correspondance des données de balayage de la tige de balayage cible avec les données standard de la tige de balayage cible.

5. Procédé d'acquisition selon la revendication 4, dans lequel l'étape consistant à acquérir des informations d'identification de la tige de balayage d'objet cible comprend :
l'acquisition d'informations d'identification de la tige de balayage d'objet cible à partir de la demande de balayage de cavité buccale.

6. Procédé d'acquisition selon la revendication 2, dans lequel l'étape consistant à mettre en correspondance les données de balayage de la tige de balayage cible avec des données standard de la tige de balayage cible comprend :
l'acquisition d'une certaine quantité de données de balayage à chaque fois dans le processus de balayage en temps réel et la mise en correspondance d'un balayage actuel avec les données standard, dans lequel les données de balayage actuel sont formées par assemblage de toutes les données de balayage acquises à un moment actuel et avant le moment actuel ;
ou la mise en correspondance de données de balayage actuel et des données standard à intervalles dans le processus de balayage en temps réel, dans lequel les données de balayage actuel sont formées par assemblage de toutes les données de balayage acquises à un moment actuel et avant le moment actuel.

7. Procédé d'acquisition selon la revendication 2, dans lequel l'étape consistant à mettre en correspondance les données de balayage de la tige de balayage cible avec des données standard de la tige de balayage cible comprend :
la mise en correspondance des données de balayage de la tige de balayage cible dans toutes les données de balayage avec les données standard de la tige de balayage cible après le balayage de toutes les données de balayage de l'objet cible.

8. Procédé d'acquisition selon l'une quelconque des revendications 2, 4, 6, 7, dans lequel l'étape consistant à mettre en correspondance les données de balayage de la tige de balayage cible avec des données standard de la tige de balayage cible comprend :
l'acquisition d'une base de données de tige de balayage, dans lequel la base de données de tige de balayage comprend des données standard d'une pluralité de types de tiges de balayage, les données standard de chaque type de tiges de balayage correspondent à des informations d'identification, les données standard de la pluralité de types de tiges de balayage comprennent les données standard de la tige de balayage cible, et les données standard de la tige de balayage cible correspondent à des informations d'identification de la tige de balayage cible ;
le chargement de la base de données de tige de balayage sur un serveur infonuagique ; et
le téléchargement des données standard de la tige de balayage cible à partir du serveur infonuagique.

9. Procédé d'acquisition selon l'une quelconque des revendications 2, 4, 6, 7, dans lequel l'étape consistant à mettre en correspondance les données de balayage de la tige de balayage cible avec des données standard de la tige de balayage cible comprend :
l'acquisition d'une base de données de tige de balayage, dans lequel la base de données de tige de balayage comprend des données standard d'une pluralité de types de tiges de balayage, les données standard de chaque type de tiges de balayage correspondent à des informations d'identification, les données standard de la pluralité de types de tiges de balayage comprennent les données standard de la tige de balayage cible, et les données standard de la tige de balayage cible correspondent à des informations d'identification de la tige de balayage cible ;
le chargement de la base de données de tige de balayage sur un disque mémoire distribuée d'un terminal actuel ; et
le téléchargement des données standard de la tige de balayage cible à partir du disque mémoire distribuée.

10. Système de commande d'un scanner dentaire, comprenant :
un scanner dentaire (21), configuré pour balayer un objet cible afin d'obtenir des données de balayage de l'objet cible ; et
un ordinateur (23), connecté au scanner dentaire (21) et configuré pour exécuter des programmes, dans lequel les programmes sont exécutés pour mettre en œuvre le procédé d'acquisition de données numériques de cavité buccale selon l'une quelconque des revendications 1 à 9.

11. Système de commande selon la revendication 10, comprenant en outre :
un serveur, connecté à l'ordinateur (23) et configuré pour stocker des données standard de tiges de balayage et pour envoyer des données standard d'une tige de balayage cible à l'ordinateur (23) lors de la réception d'une demande de mise en correspondance.

12. Appareil d'acquisition de données numériques de cavité buccale, comprenant :
une unité de réception (31), configurée pour recevoir une demande de balayage de cavité buccale ;
une unité d'acquisition (33), configurée pour acquérir des données de balayage d'un objet cible sur la base de la demande de balayage de cavité buccale, dans lequel les données de balayage comprennent des données de balayage d'une tige de balayage cible installée sur l'objet cible, l'objet cible est un objet sur lequel la tige de balayage cible est installée, et les données standard de la tige de balayage cible sont des données de conception correspondant à la tige de balayage cible ;
une unité de mise en correspondance (35), configurée pour mettre en correspondance les données de balayage de la tige de balayage cible avec des données standard de la tige de balayage cible ; et
une unité de remplacement (37), configurée pour utiliser les données standard mises en correspondance de la tige de balayage cible pour remplacer les données de balayage de la tige de balayage cible dans les données de balayage de l'objet cible, et pour obtenir les données numériques de cavité buccale de l'objet cible.

13. Dispositif électronique, comprenant :
un processeur ; et
une mémoire, configurée pour stocker des instructions exécutables du processeur,
dans lequel le processeur est configuré pour exécuter les instructions exécutables afin de mettre en œuvre le procédé d'acquisition de données numériques de cavité buccale selon l'une quelconque des revendications 1 à 9.

14. Support de stockage lisible par ordinateur, comprenant des programmes informatiques stockés, dans lequel, lorsque les programmes informatiques sont exécutés, un dispositif dans lequel se trouve le support de stockage lisible par ordinateur est commandé pour mettre en œuvre le procédé d'acquisition de données numériques de cavité buccale selon l'une quelconque des revendications 1 à 9.
